(19) Europäisches Patentamt
European Patent Office
Office européen des brevets

(11) Numéro de publication: **0 509 925 B1**

(12) **FASCICULE DE BREVET EUROPEEN**

(45) Date de publication de fascicule du brevet: **09.08.95**

(51) Int. Cl.[6]: **C07C 45/78**, C07C 45/85, C07C 45/82, C07C 49/203, C07C 45/74, C07C 49/24, C07C 45/72, C07F 5/04, C07F 19/00

(21) Numéro de dépôt: **92401069.7**

(22) Date de dépôt: **16.04.92**

(54) **Procédé de purification de la pseudo-ionone.**

(30) Priorité: **17.04.91 FR 9104711**

(43) Date de publication de la demande:
**21.10.92 Bulletin 92/43**

(45) Mention de la délivrance du brevet:
**09.08.95 Bulletin 95/32**

(84) Etats contractants désignés:
**AT BE CH DE DK ES FR GB GR IT LI LU NL PT SE**

(56) Documents cités:
**EP-A- 0 295 361**
**EP-A- 0 351 190**
**US-A- 3 161 684**

(73) Titulaire: **RHONE-POULENC NUTRITION ANIMALE**
**Rue Marcel Lingot**
**F-03600 Commentry (FR)**

(72) Inventeur: **Chabardes, Pierre**
**24 rue Jeanne d'Arc**
**F-69110 Sainte Foy les Lyon (FR)**
Inventeur: **Crenne, Noel**
**114 bis rue Hénon**
**F-69004 Lyon (FR)**

(74) Mandataire: **Le Pennec, Magali et al**
**RHONE-POULENC RORER SA,**
**Direction des Brevets,**
**20 Avenue Raymond Aron**
**F-92165 Antony Cédex (FR)**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition (art. 99(1) Convention sur le brevet européen).



Rank Xerox (UK) Business Services
(3.10/3.09/3.3.3)

## Description

La présente invention concerne un nouveau procédé de purification de la pseudo-ionone.

La pseudo-ionone est préparée à partir du citral et de l'acétone par la réaction qui peut être schématisée de la manière suivante :

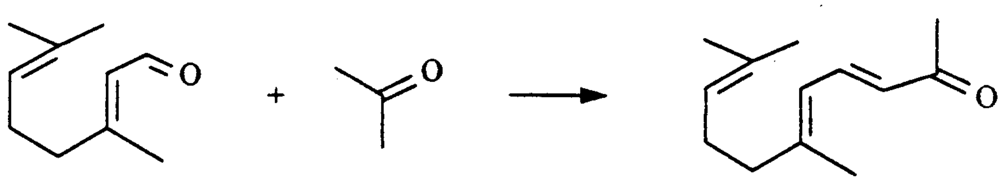

cette réaction est réalisée à une température comprise entre 0 et 100 ° C; lors de la réaction il se forme en même temps un produit secondaire de formule (I), dû à une impureté du citral qui est le produit de condensation de l'isocitral sur l'acétone selon la réaction suivante :

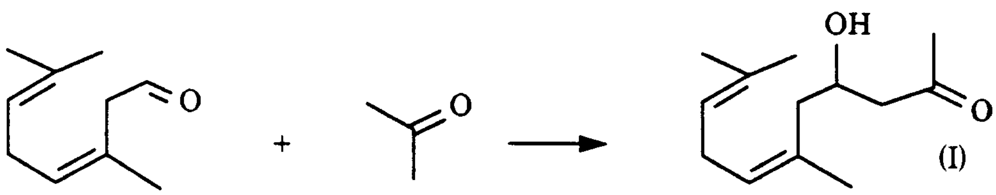

(I)

Ce produit de condensation de formule (I) se déshydrate plus difficilement que l'aldol du citral, formé intermédiairement, qui conduit à la pseudo-ionone.

Le produit de condensation de formule (I) présente en plus un point d'ébullition voisin de celui de la pseudo-ionone ce qui nécessite pour la séparation de ces deux composés l'usage d'une distillation fractionnée qui entraine une dégradation partielle de la pseudo-ionone.

Il a été découvert, un procédé simple, objet de la présente invention, qui permet au cours de l'étape d'isolement de la pseudo-ionone de s'affranchir de la présence du composé de formule (I).

Le procédé de l'invention consiste à chauffer le mélange de condensation du citral et de l'acétone contenant le composé de formule (I) en présence d'un dérivé métallique ou d'un dérivé du bore, puis à distiller la pseudo-ionone formée.

Pendant ce procédé, un complexe de formule (II) est formé entre le composé de formule (I) et le métal ou le bore

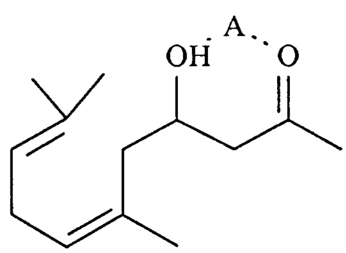

Formule (II)

où A représente le métal ou le bore.

Le composé de formule(II) ne distille pas avec la pseudo-ionone, il peut être donc séparé de la pseudo-ionone par distillation.

Les dérivés métalliques sont choisis parmi :

- les dérivés du magnésium tels que l'acétate de magnésium
- les dérivés du titane tels que le titanate d'isopropyle
- les dérivés du vanadium tels que l'ortho vanadate de triéthanolamine
- les dérivés du chrome tels que l'acétate de chrome

- les dérivés du nickel tels que l'acétate de nickel
- les dérivés du zinc tels que l'acétate de zinc
- les dérivés de l'aluminium tels que l'isopropylate d'aluminium
- les dérivés du plomb tels que l'acétate de plomb.

Les dérivés du bore sont choisis parmi l'anhydride borique, l'acide borique, le borate de n.butyle.

Ces dérivés sont éventuellement mis en oeuvre en présence d'une amine tertiaire.

On préfère parmi les dérivés cités utiliser les dérivés du titane, du bore et du magnésium.

La réaction entre le dérivé métallique et le mélange de réaction est de préférence réalisée à une température comprise entre 50 et 150 °C. La pression utilisée lors de la mise en contact du mélange réactionnel et du composé métallique ou du bore et lors de la distillation de la pseudo-ionone est de préférence comprise entre 2660 et 13300 Pa (20 et 100 mm de mercure).

Par ce procédé il est possible d'éliminer au moins 80 % du composé de formule (I) sans observer de dégradation de la pseudo-ionone.

La présente invention sera plus complètement décrite à l'aide des exemples suivants qui ne doivent pas ête considérés comme limitatifs de l'invention.

EXEMPLE 1

On chauffe la pseudo-ionone brute en présence de borate de t.butyle, à raison de 8,75 moles de borate par mole d'aldol de formule (I), de 25 °C à 105 °C en 20 minutes puis pendant 45 minutes à 120-130 °C sous pression réduite (100 mm de mercure ; 13,3 kPa).

Les déterminations analytiques montrent que tout l'aldol a disparu et que l'on récupère 99,4 % de la pseudo-ionone présente dans le produit brut.

EXEMPLE 2

On opère comme à l'exemple 1 en remplaçant le borate de t.butyle par l'acide borique à raison de 5,75 moles d'acide borique par mole d'aldol de formule (I). On chauffe de 25 à 105 °C en 35 minutes puis pendant 2 heures 10 minutes à 100-110 °C sous pression réduite (100 mm de mercure ; 13,3 kPa).

Les déterminations analytiques montrent que tout l'aldol de formule (I) a disparu et que l'on récupère la totalité de la pseudo-ionone.

EXEMPLE 3

On opère comme dans l'exemple 2 en remplaçant l'acide borique par l'anhydride borique à raison de 1,6 mole d'anhydride borique par mole d'aldol de formule (I). Après 4 heures 15 minutes de chauffage sous pression réduite (100 mm de mercure ; 13,3 kPa), les déterminations analytiques montrent que tout l'aldol de formule (I) a disparu et que l'on récupère la totalité de la pseudo-ionone.

EXEMPLE 4

On chauffe pendant 30 minutes à 90 °C la pseudo-ionone brute en présence de titanate d'isopropyle à raison de 1,94 mole de titanate d'isopropyle par mole d'aldol de formule (I). Les déterminations analytiques montrent que tout l'aldol de formule (I) a disparu et que l'on récupère la totalité de la pseudo-ionone.

EXEMPLES 5 A 15

On opère comme précédemment en chauffant la peudo-ionone brute à 130 °C sous pression réduite (40 mm de mercure ; 5,3 kPa) en présence de différents dérives métalliques :

| N°exemple | Dérivé métallique (DM) | DM/aldol (mole) | Durée du chauffage | Aldol éliminé (%) | Pseudo -ionone dégradée (%) |
|---|---|---|---|---|---|
| 5 | $Al[OCH(CH_3)_2]_3$ | 1 | 1h 45 | 80 | 3,6 |
| 6 | Titanate de triéthanolamine | 0,92 | 1h 30 | 90 | 4,6 |
| 7 | $Ni(OCOCH_3)_2,4H_2O$ | 1,086 | 1h 30 | 84 | 0 |
| 8 | $Zn(OCOCH_3)_2$ | 1 | 1h 30 | 86 | 1,2 |
| 9 | $Co(OCOCH_3)_2,4H_2O$ | 1,38 | 1h 30 | 87 | 0 |
| 10 | $Cr(OCOCH_3)_3,2H_2O$ | 1,15 | 1h 30 | 85 | 0 |
| 11 | $Mg(OCOCH_3)_2,2H_2O$ | 1,15 | 1h 30 | 100 | 0 |
| 12 | $Mn(OCOCH_3)_2,4H_2O$ | 1,4 | 1h 30 | 100 | 4,9 |
| 13 | $Pb(OCOCH_3)_2,3H_2O$ | 1,22 | 1h 30 | 100 | 6 |
| 14 | Orthovanadate de triéthanolamine | 1,06 | 1h 30 | 90 | 3,1 |
| 15 | - | - | 1h 30 | 4 | 0 |

**Revendications**

**1.** Procédé de purification de la pseudo-ionone obtenue par la réaction suivante :

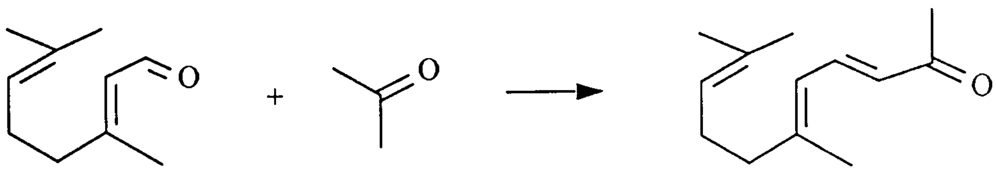

caractérisé en ce qu'on met en contact la pseudo-ionone obtenue avec un dérivé métallique choisi parmi les dérivés du magnésium, du titane, du vanadium, du chrome, du nickel, du zinc, de l'aluminium, du plomb ou un dérivé du bore puis on distille la pseudo-ionone.

**2.** Procédé selon la revendication 1 caractérisé en ce que le dérivé métallique est choisi parmi les dérivés du titane ou du magnésium.

**3.** Procédé selon la revendication 1 caractérisé en ce que le dérivé du bore est le borate de tertiobutyle, l'acide borique ou l'anhydride borique.

**4.** Procédé selon l'une quelconque des revendications précédentes caractérisé en ce que l'on ajoute une amine tertiaire.

**5.** Procédé selon l'une quelconque des revendications précédentes caractérisé en ce que la température de réaction est comprise entre 50 et 150 ° C.

**6.** Procédé selon l'une quelconque des revendications précédentes caractérisé en ce que la pression réactionnelle est comprise entre 2660 Pa et 13300 Pa (20 et 100 mm de mercure).

**7.** Procédé selon la revendication 1 caractérisé en ce que le contact est réalisé avec un mélange contenant la pseudo-ionone et un dérivé de formule (I) :

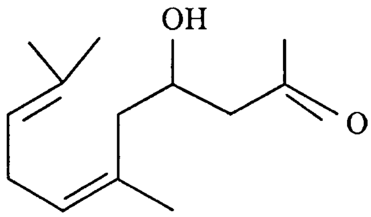

**8.** Complexe de formule

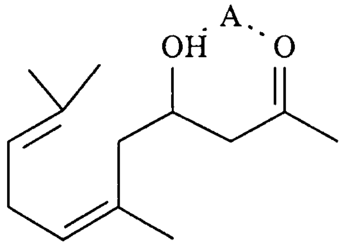

où A représente le magnésium, le titane, le vanadium, le chrome, le nickel, le zinc, le bore, l'aluminium ou le plomb.

## Claims

1. Process for the purification of the pseudo-ionone obtained by the following reaction:

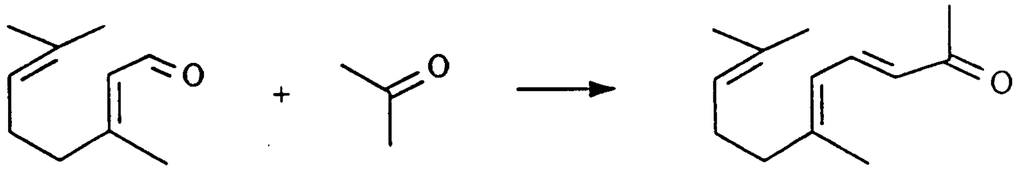

characterized in that the pseudo-ionone obtained is placed in contact with a metal derivative chosen from the magnesium, titanium, vanadium, chromium, nickel, zinc, aluminium or lead derivatives or a boron derivative, followed by distillation of the pseudo-ionone.

2. Process according to claim 1, characterized in that the metal derivative is chosen from the titanium or magnesium derivatives.

3. Process according to claim 1, characterized in that the boron derivative is tert-butyl borate, boric acid or boric anhydride.

4. Process according to any one of the preceding claims, characterized in that a tertiary amine is added.

5. Process according to any one of the preceding claims, characterized in that the reaction temperature is between 50 and 150 ° C.

6. Process according to any one of the preceding claims, characterized in that the reaction pressure is between 2660 Pa and 13,300 Pa (20 and 100 mm of mercury).

7. Process according to claim 1, characterized in that the contact is achieved with a mixture containing pseudo-ionone and a derivative of formula (I):

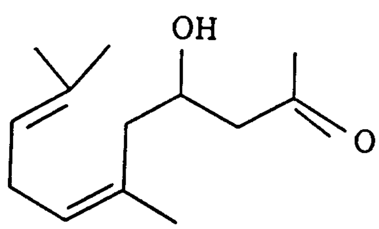

8. Complex of formula

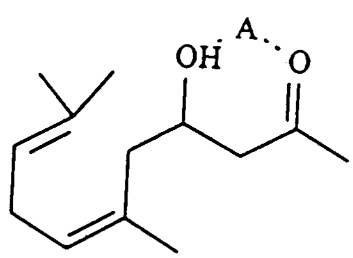

where A represents magnesium, titanium, vanadium, chromium, nickel, zinc, boron, aluminium or lead.

## Patentansprüche

1. Verfahren zur Reinigung von durch die nachstehende Reaktion erhaltenem Pseudoionon

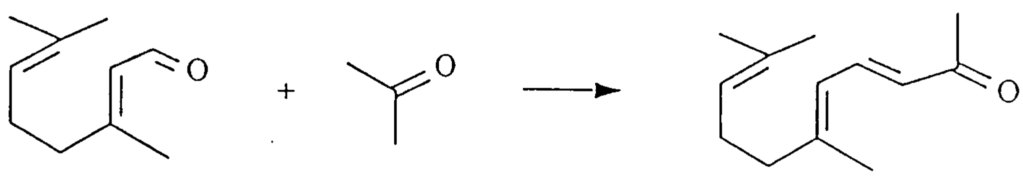

dadurch **gekennzeichnet,** daß man das erhaltene Pseudoionon mit einem Metallderivat, ausgewählt aus Magnesium-, Titan-, Vanadium-, Chrom-, Nickel-, Zink-, Aluminium-, Bleiderivaten oder einem Borderivat in Kontakt bringt, anschließend das Pseudoionon destilliert.

2. Verfahren nach Anspruch 1, dadurch **gekennzeichnet,** daß man das Metallderivat aus den Titan- oder Magnesiumderivaten auswählt.

3. Verfahren nach Anspruch 1, dadurch **gekennzeichnet,** daß das Borderivat tert.-Butylborat, Borsäure oder Borsäureanhydrid ist.

4. Verfahren nach einem der vorstehenden Ansprüche, dadurch **gekennzeichnet,** daß man ein tertiäres Amin hinzufügt.

5. Verfahren nach einem der vorstehenden Ansprüche, dadurch **gekennzeichnet**, daß die Reaktionstemperatur zwischen 50 und 150 ° C liegt.

6. Verfahren nach einem der vorstehenden Ansprüche, dadurch **gekennzeichnet**, daß der Reaktionsdruck zwischen 2660 Pa und 13300 Pa (20 und 100 mm Hg) liegt.

7. Verfahren nach Anspruch 1, dadurch **gekennzeichnet**, daß man den Kontakt mit einem Gemisch, das Pseudoionon und ein Derivat der Formel (I):

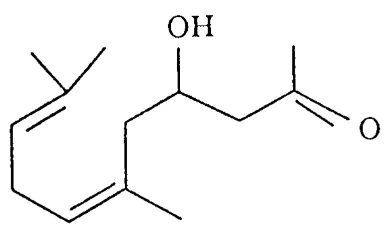

enthält, durchführt.

**8.** Komplex der Formel

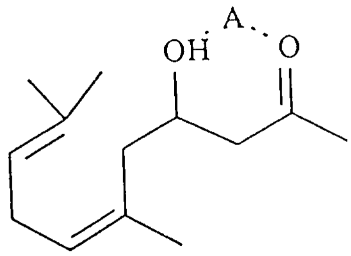

worin A für Magnesium, Titan, Vanadium, Chrom, Nickel, Zink, Bor, Aluminium oder Blei steht.